(19) Europäisches Patentamt
European Patent Office
Office européen des brevets

(11) Publication number: 0 085 717 B1

# (12) EUROPEAN PATENT SPECIFICATION

(45) Date of publication of patent specification: 29.10.86

(51) Int. Cl.[4]: C 07 J 9/00

(21) Application number: 82902934.7

(22) Date of filing: 19.08.82

(86) International application number: PCT/US82/01123

(87) International publication number: WO 83/00694 03.03.83 Gazette 83/06

## (54) NONDENATURING ZWITTERIONIC DETERGENTS FOR MEMBRANE BIOCHEMISTRY.

(30) Priority: 19.08.81 US 294203

(43) Date of publication of application: 17.08.83 Bulletin 83/33

(45) Publication of the grant of the patent: 29.10.86 Bulletin 86/44

(84) Designated Contracting States: AT BE CH DE FR GB LI LU NL SE

(56) References cited:
EP-A-0 046 523
US-A-4 207 308
US-A-4 220 598
US-A-4 264 514
CHEMICAL ABSTRACTS, vol. 95, no. 5, August 3, 1981, page 373, abstract no. 38693c COLUMBUS OHIO (US)
CHEMICAL ABSTRACTS, vol. 94, no. 11, March 16, 1981, page 345, abstract no. 79812p COLUMBUS OHIO (US)
CHEMICAL ABSTRACTS, vol. 93, no. 23, December 8, 1980, page 252, abstract no. 217549r COLUMBUS OHIO (US)
CHEMICAL ABSTRACTS, vol. 97, no. 7, August 16, 1982, page 318, abstract no. 52173f COLUMBUS OHIO (US)

(73) Proprietor: THE UNITED STATES OF AMERICA as represented by the Secretary UNITED STATES DEPARTMENT OF COMMERCE
5285 Port Royal Road
Springfield, VA 22161 (US)

(72) Inventor: HJELMELAND, Leonard Martin
8605 Maryland Avenue
Bethesda, MD 20014 (US)

(74) Representative: Kador . Klunker . Schmitt-Nilson . Hirsch
Corneliusstrasse 15
D-8000 München 5 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 085 717 B1

**Description for the Contracting States: BE CH DE FR GB LI**

This invention relates to the use of 2-hydroxy-sulfopropyl-derivatives as nondenaturing zwitter-ionic detergents. The derivative consists for example of an effective amount of 3-[(3-cholamidopropyl)-dimethylammonio]-2-hydroxy-1-propansulfonate (CHAPSO). These detergents are of extreme interest in the biological study of proteins due to the nondenaturing characteristics. Other examples of the group may be prepared from different alicyclic compounds, for example, utilizing cholic acid and in others deoxycholic acid and dehydroabietic acid.

The detergents are useful in biochemistry. They combine the useful properties of both the sulfobetaine type detergents and the bile salt anions. The new detergent proves to be effective at solubilizing membrane proteins in a non-denatured state.

The preparation of 2-hydroxy-sulfopropyl-derivatives is disclosed in the earlier European patent application EP—A—46 523. The applicant has delimited the claims over this prior art, according to article 54, No. 4 of the European patent convention and prosecutes the application with a different specification and claims in the designated contracting states: Austria, Luxemburg, Netherlands and Sweden.

EP—A—46 523 has not been considered in the assessment of inventive step (article 54(3) EPC).

Other prior art documents are:

Gonenne, et al., "Solubilization of Membrane Proteins by Sulfobetaines, Novel Zwitterionic Surfactants", *Analytical Biochemistry,* 87:28—38 (1978).

Hjelmeland, et al., "Electrofusing of Integral Membrane Proteins in Mixtures of Zwitterionic and Nonionic Detergents", *Analytical Biochemistry,* 95:201—208 (1979).

Parris, et al., "Soap-Based Detergent Formulations: XII. Alternate Syntheses of Surface Active Sulfobetaines", *J. Amer. Oil Chem. Soc.,* 53:60—62 (1976).

Parris, et al., "Soap-Based Detergent Formulations: XVIII. Effect of Structure Variations on Surface-Active Properties of Sulfur Containing Amphoteric Surfactants", *J. Amer. Oil Chem. Soc.,* 53:97—100 (1976).

Parris, et al., "Soap Based Detergent Formulation: XXIV. Sulfobetaine Derivatives of Fatty Amides", *J. Amer. Oil Chem. Soc.,* 54:294—296 (1977).

Parris, et al., "Soap based Detergent Formulations: V. Amphoteric Lime Soap Dispersing Agents", *J. Am. Oil Chem. Soc.,* 50:509—512 (1973).

Herrmann, "Micellar Properties of Some Zwitterionic Surfactants", *J. Colloid Interface Sci.,* 22:352—359 (1966).

Parris, et al., "Determination of Sulfobetaine Amphoteric Surfactants by Reverse Phase High Performance Liquid Chromatography" *Anal. Chem.* 49:2228—2231 (1977).

Konig, *Z. Anal. Chem.,* 259:191—194 (1973).

British Patent 1,037,649.

Definition

In this specification the following definition from Condensed Chemical Dictionary, *9,* page 107, is given. Bile acid and salt are defined as an acid and acid salt found in bile (the secretion of liver); bile acids are steroids having a hydroxy group and a 5-carbon atom side chain terminating in a carboxyl group; cholic acid is mentioned as the most abundant bile acid in human bile.

Introduction

One of the more important aspects of the purification of membrane proteins is the choice of a suitable detergent. This choice is usually based on the ability to preserve an enzymatic activity or some other native property. In this respect, nonionic detergents such as Triton X—100 (octylphenoxypolyethoxy ethanol; trademark of Rohm and Haas) and Lubrol PX (blend of nonyl phenol and ethylene oxide; trademark of ICI), and the bile salts are the detergents of choice. Two additional considerations must also be made. The first relates to the artificial aggregation of proteins while in the presence of detergents to form nonspecific protein complexes which have no biological relevance. A useful detergent should be capable of breaking such interactions to give maximally disaggregated species in solution. Nonionics are generally less efficient in this respect than are ionic detergents or bile salt anions. The second consideration is the extent to which the detergent affects the charge properties of solubilized proteins. Anionic detergents, for example, add substantial amount of negative charge which may completely overshadow the charge properties of the native protein. This type of charge alteration profoundly affects the utility of conventional techniques such as ion exchange chromatography and isoelectric focusing which depend primarily on charge properties to effect protein separations.

A survey of existing detergents demonstrates that no single compound which is presently available is adequately nondenaturing, disaggregating, and at the same time electrically neutral. The bile salts are both nondenaturing and effective in disaggregating protein but lack the charge neutrality necessary for compatibility with charge fractionation techniques. In contrast, Triton X—100 and other polyethoxy-type nonionics are electrically neutral and nondenaturing but appear not to be efficient at breaking protein-protein interactions. N-alkyl sulfobetaines are neutral and efficient at disaggregating protein but are unfortunately strongly denaturing. One possibility of a detergent useful in the purification of membrane

proteins is a combination of a bile salt hydrophobic group and a sulfobetaine type polar group. This invention evaluates the potential utility of a sulfobetaine derivative of cholic acid as a nondenaturing zwitter-ionic detergent, example given its utility in membrane protein purification.

The present invention concerns the use of 2-hydroxy-sulfopropyl-derivatives made by reacting an acid selected from one member of a group of carboxylic acids consisting of cholic acid, deoxycholic acid and dehydroabietic acid with a polyethyleneamine selected from one member of the group consisting of

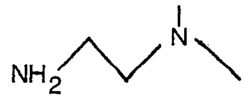 (1)

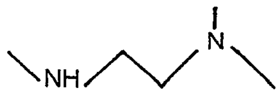 (2)

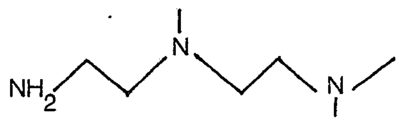 (3)

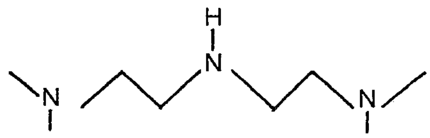 (4)

or reacted with a polypropyleneamine selected from one member of the group consisting of

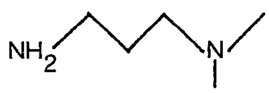 (1)

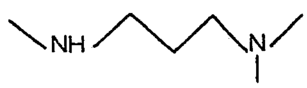 (2)

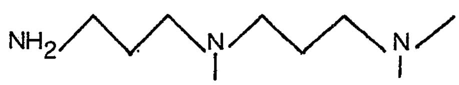 (3)

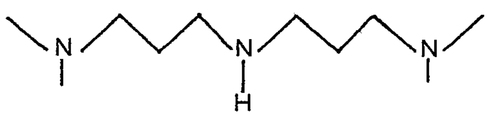 or (4)

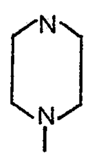

and reacting with sodium 1-chloro-2-hydroxy-3-propanesulfonate as a non-denaturing zwitter-ionic detergent.

The synthesis of 2-hydroxy-sulfopropyl-derivatives used according to the invention is disclosed in EP—A—00 46523, so that reference is made thereto.

Specific operative compounds are:

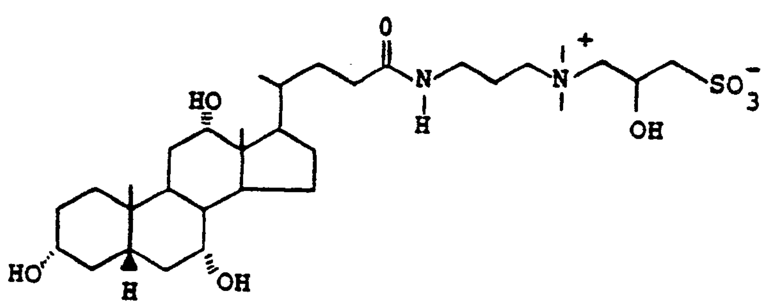 (CHAPSO) (1)

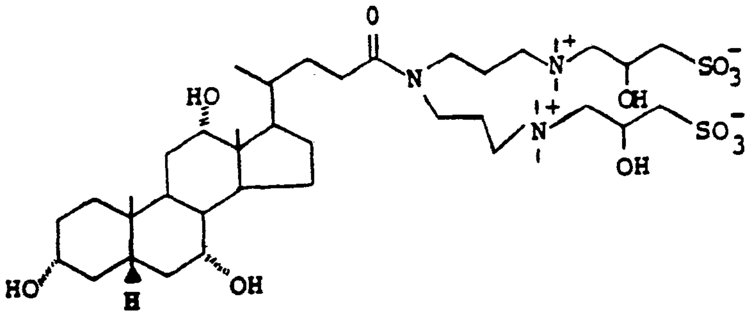

(2)

Comparison of CHAPSO relative to CHAPS

In an evaluation of 3-(3-cholamidopropyl)-dimethylammonio-2-hydroxy-1-propanesulfonate (CHAPSO) relative to CHAPS in the opiate receptor solubilization assay, solubilization and Sepharose 6B chromatography of active opiate receptors was performed as described by Simonds, et al., in Proc. Nat. Acad. Sci. U.S.A., 77, 4623—4627 (1980). The Figure demonstrates that 3[(3-cholamidopropyl)-dimethylammonio]-2-hydroxy-1-propanesulfonate (CHAPSO) is about twice as effective as CHAPS at solubilizing active opiate receptors. CHAPS is 3-(3-cholamidopropyl)-dimethylammonio-1-propanesulfonate. The advantages obtained by CHAPS are extensively discussed in EP—A—00 46 523, so that reference is made thereto.

**Description for the Contracting States: AT LU NL SE**

This invention relates to a process for producing nondenaturing zwitterionic detergents for proteins, particularly for use in membrane biochemistry and to compounds obtained according to this process. Such nondenaturing zwitterionic detergents are known (Chemical Abstracts, vol. 95, no. 5, 1981, page 373, abstract no. 38693c; vol. 94, no. 11, 1981, page 345, abstract no. 79812p; and vol. 93, no. 23, 1980, page 252, abstract no. 217459r) and consist for example of an effective amount of 3-(3-cholamidopropyl)-dimethyl-ammonio-1-propanesulfonate (CHAPS). This detergent is of extreme interest in the biological study of proteins due to its nondenaturing characteristic.

These detergents combine the useful properties of both the sulfobetaine type detergents and the bile salt anions and prove to be effective at solubilizing membrane proteins in a nondenatured state.

Further prior art references are:

Gonenne, et al., "Solubilization of Membrane Proteins by Sulfobetaines, Novel Zwitterionic Surfactants", *Analytical Biochemistry,* 87:28—38 (1978).

Hjelmeland, et al., "Electrofocusing of Integral Membrane Proteins in Mixtures of Zwitterionic and Nonionic Detergents", *Analytical Biochemistry,* 95:201—208 (1979).

Parris, et al., "Soap-Based Detergent Formulations: XII. Alternate Syntheses of Surface Active Sulfobetaines", *J. Amer. Oil Chem. Soc.,* 53:60—63 (1976).

Parris, et al., "Soap-Based Detergent Formulations: XVIII. Effect of Structure Variations on Surface-Active Properties of Sulfur Containing Amphoteric Surfactants", *J. Amer. Oil Chem. Soc.,* 53:97—100 (1976).

Parris, et al., "Soap Based Detergent Formulation: XXIV. Sulfobetaine Derivatives of Fatty Amides", *J. Amer. Oil Chem. Soc.,* 54:294—296 (1977).

Parris, et al., "Soap Based Detergent Formulations: V. Amphoteric Lime Soap Dispersing Agents", *J. Amer. Oil Chem. Soc.,* 50:509—512 (1973).

Herrmann, "Micellar Properties of Some Zwitterionic Surfactants", *J. Colloid Interface Sci.,* 22:352—359 (1966).

Parris, et al., "Determination of Sulfobetaine Amphoteric Surfactants by Reverse Phase High Performance Liquid Chromatography" *Anal. Chem.* 49:2228—2231 (1977).

Konig, *Z. Anal. Chem.*, 259:191—194 (1972).

British Patent 1,037,645.

The applicant has filed a different specification and claims being delimited over EP—A—46 523 for the designated countries: CH, LI, DE, FR, GB, BE. EP—A—46 523 is irrelevant for the states Austria, Luxembourg, Netherlands and Sweden.

In this specification the following definition from Condensed Chemical Dictionary, *9,* page 107, is given. Bile acid and salt are defined as an acid and acid salt found in bile (the secretion of liver); bile acids are steroids having a hydroxy group and a 5-carbon atom side chain terminating in a carboxyl group; cholic acid is mentioned as the most abundant bile acid in human bile.

Introduction

One of the more important aspects of the purification of membrane proteins is the choice of a suitable detergent. This choice is usually based on the ability to preserve an enzymatic activity or some other native property. In this respect, nonionic detergents such as Triton X—100 (octylphenoxy polyethoxy ethanol; Rohm and Haas) and Lubrol PX (blend of nonyl phenol and ethylene oxide; ICI), and the bile salts are the reagents of choice. Two additional considerations must also be made. The first relates to the artificial aggregation of proteins while in the presence of detergents to form nonspecific protein complexes which have no biological relevance. A useful detergent should be capable of breaking such interactions to give maximally disaggregated species in solution. Nonionics are generally less efficient in this respect than are ionic detergents or bile salt anions. The second consideration is the extent to which the detergent affects the charge properties of solubilized proteins. Anionic detergents, for example, add substantial amounts of negative charge which may completely overshadow the charge properties of the native protein. This type of charge alteration profoundly affects the utility of conventional techniques such as ion exchange chromatography and isoelectric focusing which depend primarily on charge properties to effect protein separations.

A survey of existing detergents demonstrates that no single compound which is presently available is adequately nondenaturing, disaggregating, and at the same time electrically neutral. The bile salts are both nondenaturing and effective in disaggregating protein but lack the charge neutrality necessary for compatibility with charge fractionation techniques. In contrast, Triton X—100 and other polyethoxy-type nonionics are electrically neutral and nondenaturing but appear not to be efficient at breaking protein-protein interactions. N-alkyl sulfobetaines are neutral and efficient at disaggregating protein but are unfortunately strongly denaturing. One possibility of a detergent useful in the purification of membrane proteins is a combination of a bile salt hydrophobic group and a sulfobetaine type polar group. This invention describes the synthesis and properties of a sulfobetaine derivative of cholic acid and evaluates its potential utility in membrane protein purification.

The figure shows an evaluation of 3-[(3-cholamidopropyl)dimethylammonio]-2-hydroxy-1-propanesulfonate with a 2:1 advantage over CHAPS in solubilizing active opiate receptors.

Generalized Procedure

Below is a step-by-step outline of the synthesis described in this invention.

Step 1. The triethylammonium salt of cholic acid is formed in THF.

Step 2. After the salt is completely dissolved in THF, ethyl chloroformate is added and the flask is cooled to 0°C. At this point, a precipitate is formed which is triethylamine hydrochloride. This is filtered away from the mixed anhydride.

Step 3. The mixed anhydride then reacts with the dimethylaminopropylamine to form a dimethylaminopropyl derivative of a carboxylic acid amide, ethanol, and carbon dioxide as a gas.

Step 4. In the final step, the tertiary amine group is reacted with sodium 1-chloro-2-hydroxy-3-propanesulfonate to give the final product.

The carboxylic acids may be:

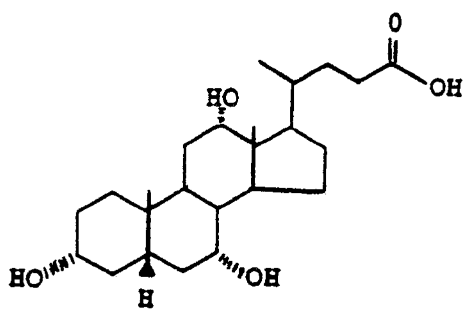

Cholic Acid .

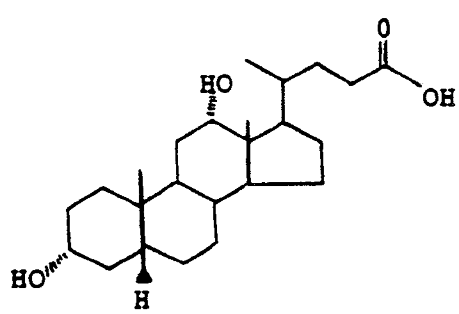

Deoxycholic Acid

or

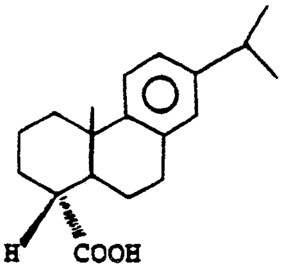

Dehydroabietic Acid

The dehydroabietic acid is important since it represents the most abundant naturally occurring group organic acids, the rosin acids which are refined from wood rosin and tall oil and have wide industrial uses, among them, the synthesis of surfactants.

The polyamines may be:

*Polyethyleneamines*

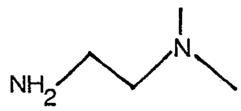

(1)

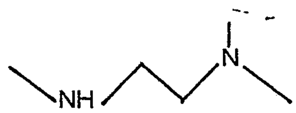

(2)

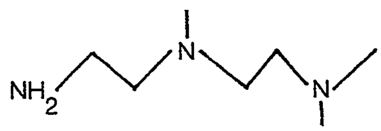

(3)

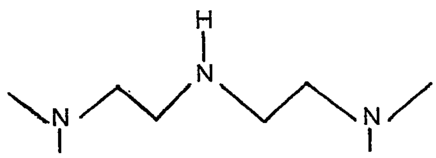

(4)

or

*Polypropyleneamines*

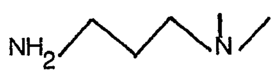

(1)

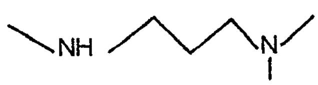

(2)

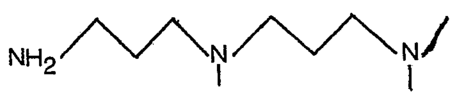

(3)

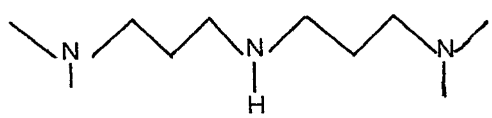

(4)

*Others* — such as N-methyl piperazine

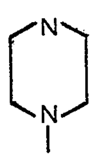

The alkylating agent is:

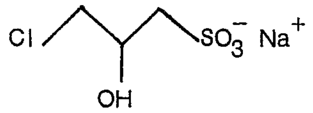 Sodium 3-Cl, 2-OH-Propanesulfonate,

Specific operative compounds are:

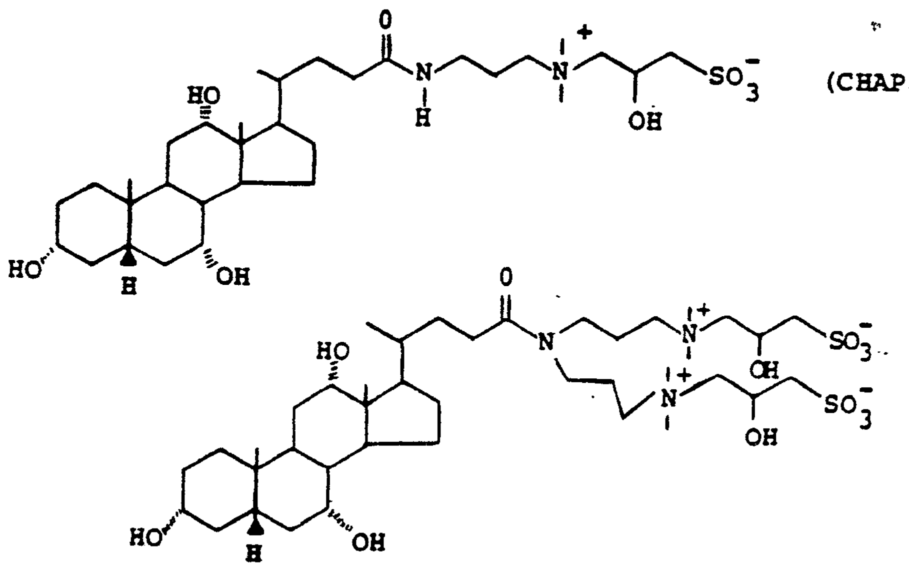

(CHAPSO) (1)

(2)

Example 1

Synthesis of 3-[(3-cholamidopropyl)dimethylammonio]-2-hydroxy-1-propanesulfonate

N-(3-dimethylaminopropyl)cholamide was prepared as described in the parent patent application U.S. Serial No. 181,465). Sodium 1-chloro-2-hydroxy-3-propanesulfonate was prepared according to the method described by Parris et al., *J. Amer. Oil Chem. Soc., 53,* 60—63 (1976).

To a solution of 0.1 mole (49.3 g) of N-(3-dimethylaminopropyl)cholamide in 300 ml of 40% aqueous methanol was added 0.1 mole (15.15 g) of sodium 1-chloro-2-hydroxy-3-propanesulfonate. The mixture was refluxed with stirring for 3 hours, after which the solution was allowed to cool to room temperature and 100 g of RG—501 X8 mixed bed ion-exchange resin (Bio Rad) were added. The suspension of ion-exchange resin was gently stirred until the pH of the supernatant was approximately 7. The mixed bed ion-exchange resin was then filtered off and the solvent removed by distillation under reduced pressure to leave approximately 30 grams of 3-[(3-cholamidopropyl)dimethylammonio]-2-hydroxy-1-propanesulfonate (50% theory). This material was judged to be homogeneous by thin layer chromatography on Silica Gel G in a 95% methanol/5% ammonium hydroxide solvent system followed by iodine visualization.

Calculated analysis for: $C_{32}H_{58}N_2SO_8 \cdot 1H_2O$ was: C, 59.22%; H, 9.32%; N, 4.31%; S, 4.94. The experimental analysis found: C, 59.12%; H, 9.31%; N, 4.31%; S, 4.99%. Suggested trivial name CHAPSO.

In an evaluation of 3-[(3-cholamidopropyl)dimethylammonio]-2-hydroxy-1-propanesulfonate relative to CHAPS in the opiate receptor solubilization assay, solubilization and Sepharose 6B chromatography of active opiate receptors was performed as described by Simonds, et al., in *Proc. Nat. Acad. Sci. U.S.A., 77,* 4623—4627 (1980). The figure demonstrates that 3-[(3-cholamidopropyl)-dimethylammonio]-2-hydroxy-1-propanesulfonate (CHAPSO) is about twice as effective as CHAPS at solubilizing active opiate receptors.

CHAPS itself may be prepared using as analogous alkylating agent sodium 1-chloro-propanesulfonate.

**Claim for the Contracting States: BE CH DE FR GB LI**

Use as nondenaturing zwitterionic detergents of 2-hydroxy-sulfopropyl-derivatives made by reacting an acid selected from one member of a group of carboxylic acids consisting of cholic acid, deoxycholic acid and dehydroabietic acid with a polyethyleneamine selected from one member of the group consisting of

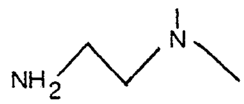

(1)

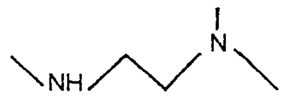

(2)

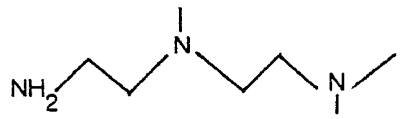

(3)

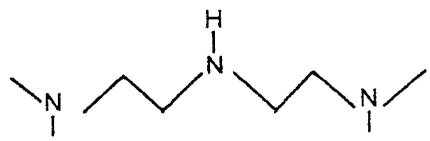

(4)

or reacted with a polypropyleneamine selected from one member of the group consisting of

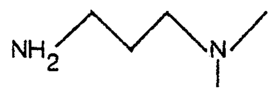

(1)

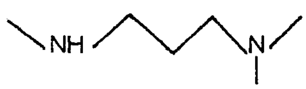

(2)

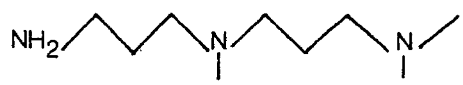

(3)

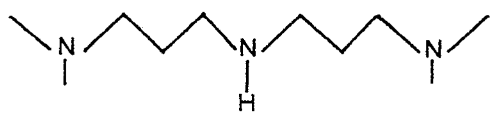

or

(4)

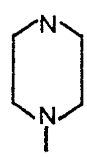

and reacting with sodium 1-chloro-2-hydroxy-3-propanesulfonate as a nondenaturing zwitter-ionic detergent.

**Claims for the Contracting States: AT LU NL SE**

1. A process for producing a nondenaturing zwitterionic detergent for protein comprising reacting an acid selected from one member of a group of carboxylic acids consisting of cholic acid, deoxycholic acid, and dehydroabietic acid reacted with a polyethyleneamine selected from one member of the group consisting of

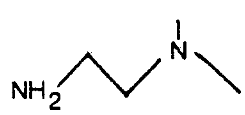

(1)

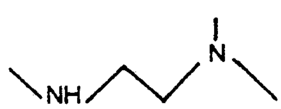

(2)

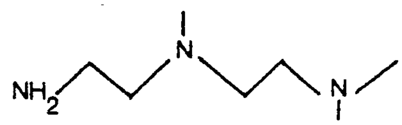

(3)

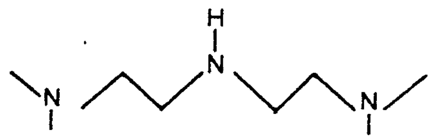

(4)

or reacted with a polypropyleneamine selected from one member of the group consisting of

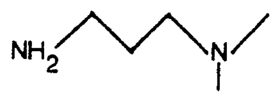

(1)

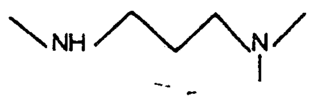

(2)

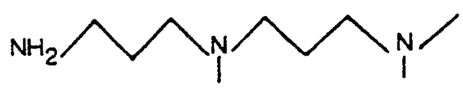

(3)

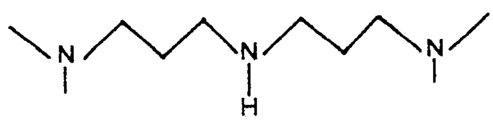

or

(4)

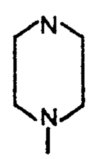

and reacting with sodium 1-chloro-2-hydroxy-3-propanesulfonate.

2. The process for producing a nondenaturing zwitterionic detergent for protein of Claim 1 comprising:

(1) reacting cholic acid with an amine to form triethylammonium salt of cholic acid in the solvent tetrahydrofuran;

(2) adding ethylchloroformate after the salt is completely dissolved and cooling the flask to 0°C and forming a precipitate which is triethylamine hydrochloride which is filtered away from the mixed anhydride;

(3) reacting the product, mixed anhydride, with a polyamine to form the polyamine derivative of a carboxylic acid amide as well as ethanol and carbon dioxide by products;

(4) reacting the polyamine with sodium 1-chloro-2-hydroxy-3-propanesulfonate.

3. The process according to Claim 1 or 2 of producing 3-[(3-cholamidopropyl)dimethylammonio]-2-hydroxy-1-propanesulfonate.

4. The compound 3-[(3-cholamidopropyl)dimethylammonio]-2-hydroxy-1-propanesulfonate produced according to the process of Claim 1 or 2.

**Patentanspruch für die Vertragsstaaten: CH LI DE FR GB BE**

Verwendung von 2-Hydroxy-sulfopropyl-Dervaten als nichtdenaturierende, zwitterionogene Detergentien, die durch Reaktion einer Säure, ausgewählt aus einem Mitglied einer Gruppe von Carboxylsäuren, die aus Cholsäure, Deoxycholsäure und Dehydroabietinsäure besteht, mit einem Polyethylenamin, ausgewählt aus einem Mitglied der Gruppe, bestehend aus

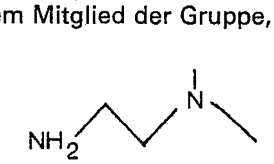

(1)

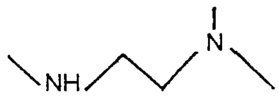

(2)

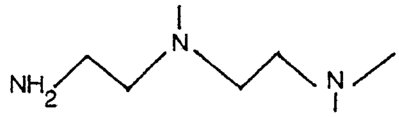

(3)

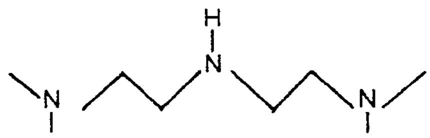

(4)

oder umgesetzt mit einem Polypropylenamin, ausgewählt aus einem Mitglied der Gruppe, bestehend aus

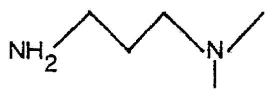

(1)

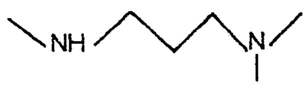

(2)

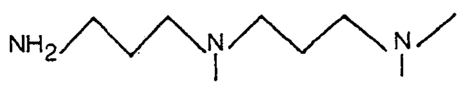

(3)

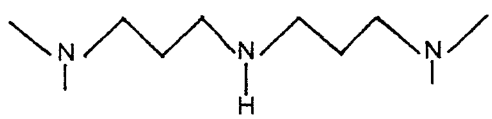

oder

(4)

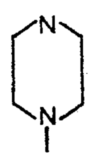

und durch Reaktion mit Natrium-1-chlor-2-hydroxy-3-propansulfonat hergestellt wird.

**Patentansprüche für die Vertragsstaaten: AT LU NL SE**

1. Verfahren zur Herstellung eines nichtdenaturierenden, zwitterionogenen Detergens für Protein, welches die Reaktion einer Säure, ausgewählt aus einem Mitglied einer Gruppe von Carboxylsäuren, die aus Cholsäure, Deoxycholsäure und Dehydroabietinsäure besteht, umgesetzt mit einem Polyethylenamin, ausgewählt aus einem Mitglied der Gruppe, bestehend aus

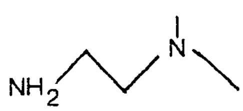

(1)

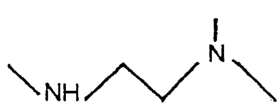

(2)

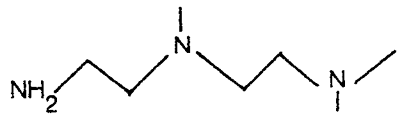

(3)

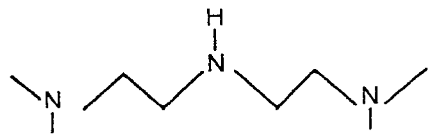

(4)

oder umgesetzt mit einem Polypropylenamin, ausgewählt aus einem Mitglied der Gruppe, bestehend aus

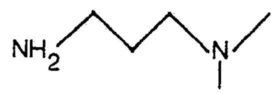

(1)

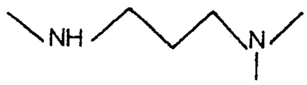

(2)

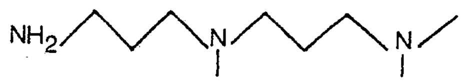

(3)

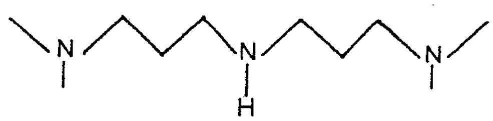

oder

(4)

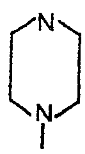

und die Reaktion mit Natrium-1-chlor-2-hydroxy-3-propansulfonat umfaßt.

2. Verfahren zur Herstellung eines nichtdenaturierenden, zwitterionogenen Detergens für Protein nach Anspruch 1, welches unfaßt:

(1) Reaktion von Cholsäure mit einem Amin in Lösungsmittel Tetrahydrofuran, um Triethylammoniumsalz von Cholsäure zu bilden;

(2) Zugabe von Ethylchloroformat, nachdem das Salz völlig aufgelöst ist, und Kühlen des Kolbens auf 0°C und Bildung eines Niederschlages, der Triethylaminhydrochlorid ist, der aus dem gemischten Anhydrid abfiltriert wird;

(3) Reaktion des Produktes, gemischtes Anhydrid, mit einem Polyamin, um das Polyamin-Derivat eines Carboxylsäureamid genauso wie Ethanol- und Kohlendioxid-Nebenprodukte zu bilden;

(4) Reaktion des Polyamins mit Natrium-1-chlor-2-hydroxy-3-propansulfonat.

3. Verfahren nach Anspruch 1 oder 2 zur Herstellung von 3-[(3-Cholamidopropyl)dimethylammonio]-2-hydroxy-1-propansulfonat.

4. Verbindung 3-[(3-Cholamidopropyl)-dimethylammonio]-2-hydroxy-1-propansulfonat, hergestellt nach dem Verfahren nach Anspruch 1 oder 2.

**Revendication pour les Etats contractants: CH LI DE FR GB BE**

Utilisation à titre de détergents amphotères non-dénaturants de dérivés 2-hydroxy-sulfopropyliques obtenus par réaction d'un acide choisi dans le groupe comprenant des acides carboxyliques constitués par l'acide cholique, l'acide désoxycholique et l'acide déhydroabiétique, avec une polyéthylèneamine choisie parmi les éléments du groupe comprenant

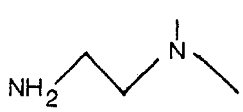

(1)

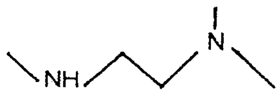

(2)

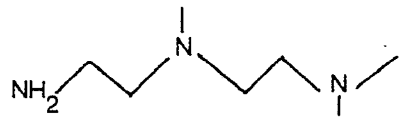

(3)

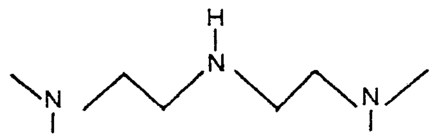

(4)

ou avec une polypropylèneamine choisie parmi les éléments du groupe comprenant

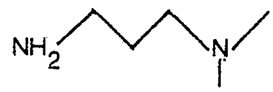

(1)

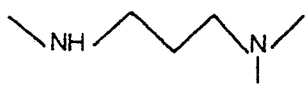

(2)

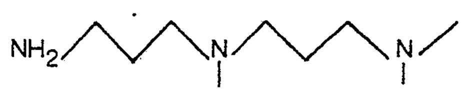

(3)

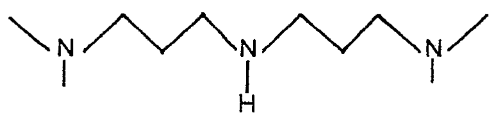

ou

(4)

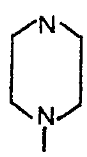

et par réaction avec le 1-chloro-2-hydroxy-3-propanesulfonate de sodium.

**Revendications pour les Etats contractants: AT LU NL SE**

1. Procédé de préparation d'un detergent amphotère non-dénaturant pour protéine qui comprend la réaction d'un acide choisi dans le groupe des acides carboxyliques constitués par l'acide cholique, l'acide désoxycholique et l'acide déhydroabiétique, avec une polyéthylèneamine choisie parmi les éléments du groupe comprenant

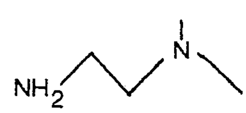

(1)

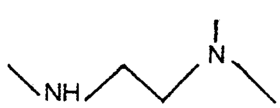

(2)

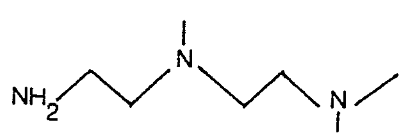

(3)

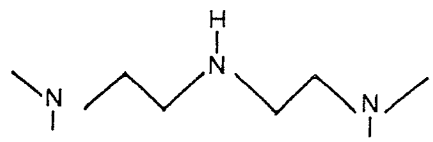

(4)

ou avec une polypropylèneamine choisie parmi les éléments du groupe comprenant

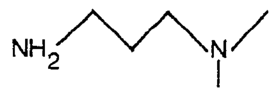

(1)

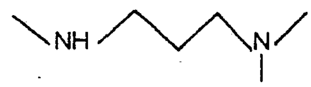

(2)

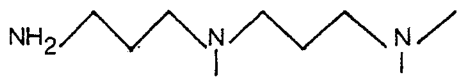

(3)

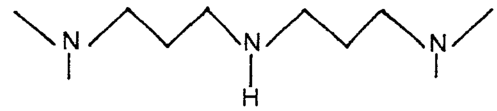

ou

(4)

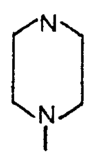

et la réaction avec le 1-chloro-2-hydroxy-3-propanesulfonate de sodium.

2. Procédé de préparation d'un détergent amphotère non-dénaturant pour protéine selon la revendication 1, qui comprend:

(1) la réaction de l'acide cholique avec une amine pour former le sel triéthylammonium de l'acide cholique dans le solvant tétrahydrofurane;

(2) l'addition de chloroformiate d'éthyle après dissolution complète du sel et refroidissement du réacteur à 0°C et formation d'un précipité constitué par le chlorhydrate de triéthylamine qui est séparé de l'anhydride mixte par filtration;

(3) la réaction du produit obtenu, l'anhydride mixte, avec une polyamine pour former le dérivé de polyamine constitué par un amide d'acide carboxylique, ainsi que de l'éthanol et du dioxyde de carbone;

(4) la réaction de la polyamine avec le 1-chloro-2-hydroxy-3-propanesulfonate de sodium.

3. Procédé selon la revendication 1 ou 2 pour la préparation du 3-[(3-cholamidopropyl)-diméthylammonio]-2-hydroxy-1-propanesulfonate.

4. Le 3-[(3-cholamidopropyl)diméthylammonio]-2-hydroxy-1-propanesulfonate préparé conformément au procédé selon la revendication 1 ou 2.

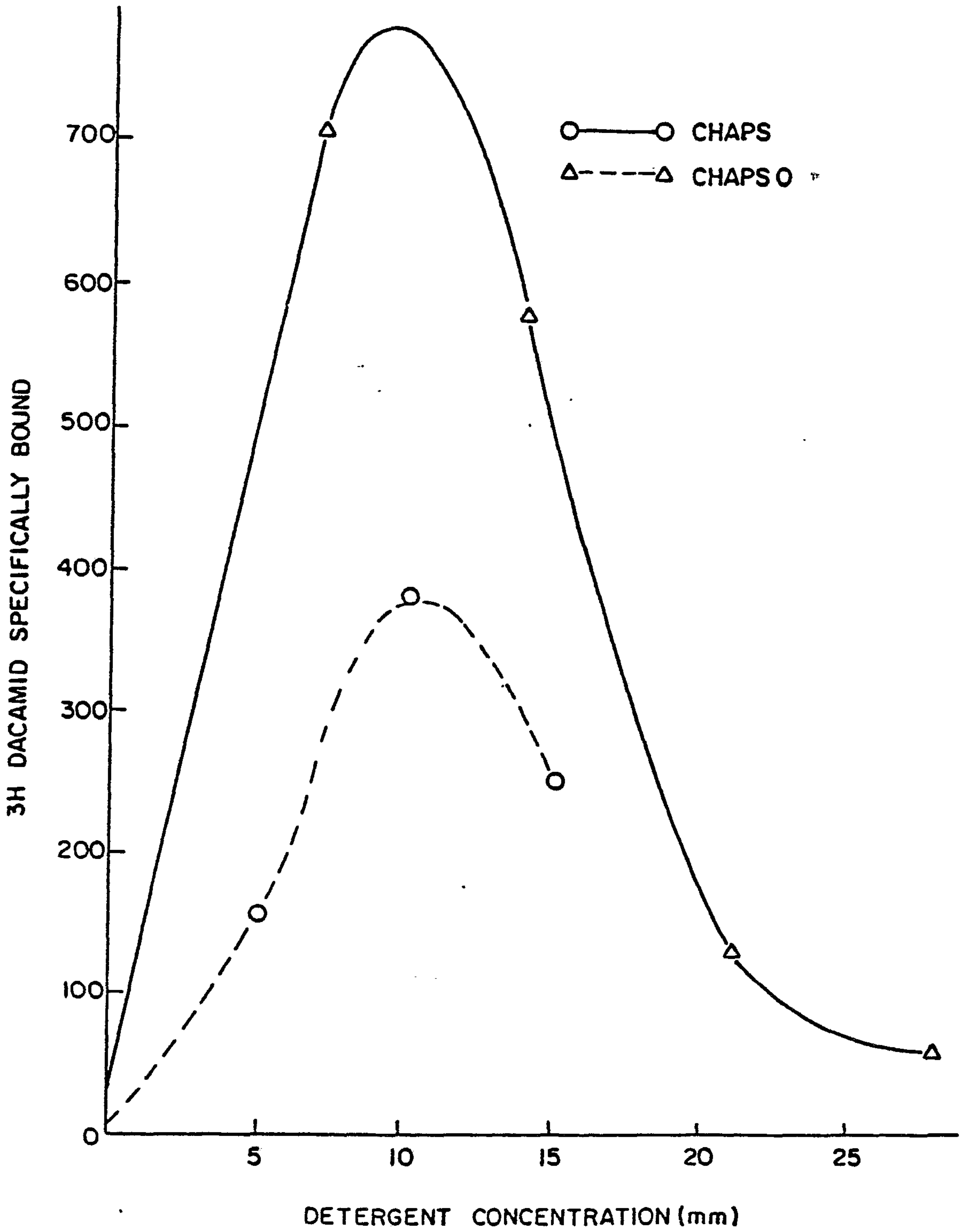
CHAPS
CHAPS O
3H DACAMID SPECIFICALLY BOUND
700
600
500
400
300
200
100
0
5
10
15
20
25
DETERGENT CONCENTRATION (mm)